# EUROPEAN PATENT APPLICATION

(11) **EP 1 958 603 A2**
(43) Date of publication of application: **20.08.2008**
(21) Application number: 07253927.3
(22) Date of filing: 03.10.2007
(51) Int. Cl.: A61F 13/15

(54) **Nursing pad**

(30) Priority: 15.02.2007 US 706688
(71) Applicant: Tyco Healthcare Retail Services AG, 8201 Schaffhausen (CH)
(72) Inventor: Thornton, Kelly, Bensalem, Pennsylvania 19020 (US); Muniz, Mariela, West Chester, Pennsylvania 19380 (US); Soto, Arginnys, Yardley, Pennsylvania 19067 (US); Bhattacharya, Neal, Conshohocken, Pennsylvania 19428 (US); Morrell-Schwartz, Linda, Bensalem, Pennsylvania 19020 (US)
(74) Representative: Elsy, David

(57) **Abstract**

A disposable nursing pad comprising an absorbent assembly having an inner surface and an outer surface with an absorbent material therebetween. The inner and outer surfaces are connected about a perimeter of the absorbent assembly which may have a non-circular shape. The absorbent assembly has a cupped configuration such that the inner surface is concave and the outer surface is convex. The absorbent pulp material may be formed with a reduced material thickness in at least one slimmer area within the absorbent assembly perimeter that is less than a total area within the absorbent assembly perimeter such that at least two areas having pulp material of differing thickness are defined.

## Description

### BACKGROUND OF THE INVENTION

### 1. Technical Field

The present invention relates to an absorbent article, and more particularly, in at least one aspect, to a nursing pad for absorbing fluid from a woman's breast.

### 2. Description of the Related Art

During lactation, it is common for breast milk to periodically leak from a woman's breasts. To prevent the milk from saturating the woman's clothing, many women utilize pads which absorb the milk which are called nursing pads.

Nursing pads are well known in the art. The pads are typically formed from multiple layers and may be either flat or shaped, such as conical shaped. Known pads are associated with problems such as bulkiness, flatness, inability to change their shape when utilized, failure to fit snugly against the woman's body when used, leakage, and/ or puckering.

Accordingly, there remains a need for an improved nursing pad that overcomes one or more or all of the foregoing problems.

### SUMMARY OF THE INVENTION

The present invention relates to a disposable absorbent article which in at least one exemplary embodiment is a nursing pad comprising an absorbent assembly having an inner surface and an outer surface with an absorbent material therebetween. The inner and outer surfaces are connected about a perimeter of the absorbent assembly with the perimeter having a non-circular shape. The absorbent assembly has a cupped configuration such that the inner surface is concave and the outer surface is convex.

In at least one exemplary embodiment, the absorbent assembly perimeter has an elliptical shape with major and minor axes wherein the ratio of the major axis to the minor axis is approximately 1.19:1.

In at least one exemplary embodiment, the absorbent assembly perimeter has a shape with an arcuate portion and opposed side portions which taper from the arcuate portion to a point. In such embodiment, the absorbent assembly perimeter may be substantially symmetrical about a plane extending between the point and a midpoint of the arcuate portion.

In at least one exemplary embodiment, the absorbent assembly perimeter has a generally triangular shape. In such embodiment, the absorbent assembly perimeter may include three equilateral sides and may further define three vertices, each of the vertices having a rounded configuration.

The absorbent article in at least one exemplary embodiment comprises a nursing pad having an absorbent assembly with an inner surface and an outer surface which are connected about a perimeter of the absorbent assembly and an absorbent pulp material provided between the inner and outer surfaces within the absorbent assembly perimeter. The absorbent pulp material is formed with a reduced material thickness in at least one slimmer area within the absorbent assembly perimeter that is less than a total area within the absorbent assembly perimeter such that at least two areas having pulp material of differing thickness are defined.

In at least one exemplary embodiment, the absorbent assembly has a center area within the absorbent assembly perimeter and the at least one slimmer area extends circumferentially about the center area such that the center area has a pulp thickness which is larger than the pulp thickness of the remainder of the area within the absorbent assembly perimeter.

In at least one exemplary embodiment, the absorbent assembly has a center area within the absorbent assembly perimeter and at least one embossing line section extends radially about the center area.

In at least one exemplary embodiment, the absorbent assembly has first and second substantial hemispheres and wherein the at least one slimmer area is in the first substantial hemisphere such that the first substantial hemisphere has a pulp thickness which is less than the pulp thickness in the second substantial hemisphere. In such an embodiment, the first and second substantial hemispheres may be divided by an arcuate contour line. A plurality of embossing line sections may be provided along the arcuate contour line.

In at least one exemplary embodiment, the article is a nursing pad having an absorbent assembly with an inner surface and an outer surface with an absorbent material therebetween. The inner and outer surfaces are connected about a perimeter of the absorbent assembly. In an unfolded condition, the absorbent assembly has a cupped configuration such that the inner surface is concave and the outer surface is convex. In a folded condition, the absorbent assembly defines opposed folded sides extending between the perimeter with the opposed folded sides extending at an angle relative to one another in the range of approximately 150° to 175°.

### BRIEF DESCRIPTION OF THE DRAWINGS

The invention is best understood from the following detailed description when read in connection with the accompanying drawings. It is emphasized that, according to common practice, the various features of the drawings are not to scale. On the contrary, the dimensions of the various features may be arbitrarily expanded or reduced for clarity. Included in the drawings are the following figures:
Fig. 1 is a perspective view of a first exemplary embodiment of an absorbent article according to aspects of this invention.
Fig. 2 is a front elevation view of the absorbent article of Fig. 1.
Fig. 2A is a front view of the absorbent article of Fig. 1 folded along its major axis.
Fig. 3 is a side elevation view of the absorbent article of Fig. 1.
Fig. 4 is a rear elevation view of the absorbent article of Fig. 1.
Fig. 5 is a sectional view along the line 5-5 in Fig. 1.
Fig. 6 is a perspective view of an absorbent article that is an alternative exemplary embodiment.
Fig. 7 is a front elevation view of the absorbent article of Fig. 6.
Fig. 8 is a side elevation view of the absorbent article of Fig. 6.
Fig. 9 is a rear elevation view of the absorbent article of Fig. 6.
Fig. 10 is a perspective view of an absorbent article that is another alternative exemplary embodiment.
Fig. 11 is a front elevation view of the absorbent article of Fig. 10.
Fig. 12 is a side elevation view of the absorbent article of Fig. 10.
Fig. 13 is a rear elevation view of the absorbent article of Fig. 10.
Fig. 14 is a perspective view of an absorbent article that is yet another alternative exemplary embodiment.
Fig. 15 is a front elevation view of the absorbent article of Fig. 14.
Fig. 16 is a side elevation view of the absorbent article of Fig. 14.
Fig. 17 is a rear elevation view of the absorbent article of Fig. 14.
Fig. 18 is a sectional view along the line 18-18 in Fig. 14.
Fig. 19 is a rear elevation view of an absorbent article that is yet another alternative exemplary embodiment.
Fig. 20 is a cross sectional view of an absorbent article that is another alternative exemplary embodiment.
Fig. 21 is a rear elevation view of an absorbent article that is yet another alternative exemplary embodiment.
Fig. 22 is a rear elevation view of an absorbent article that is another alternative exemplary embodiment.

### DETAILED DESCRIPTION OF THE INVENTION

Although the invention is illustrated and described herein with reference to specific embodiments, the invention is not intended to be limited to the details shown. Rather, various modifications may be made in the details within the scope and range of equivalents of the claims and without departing from the invention.

In the figures, the "rear" is the side of the nursing pad facing the wearer's breast and the "front" is the side facing away from the wearer toward the wearer's garment.

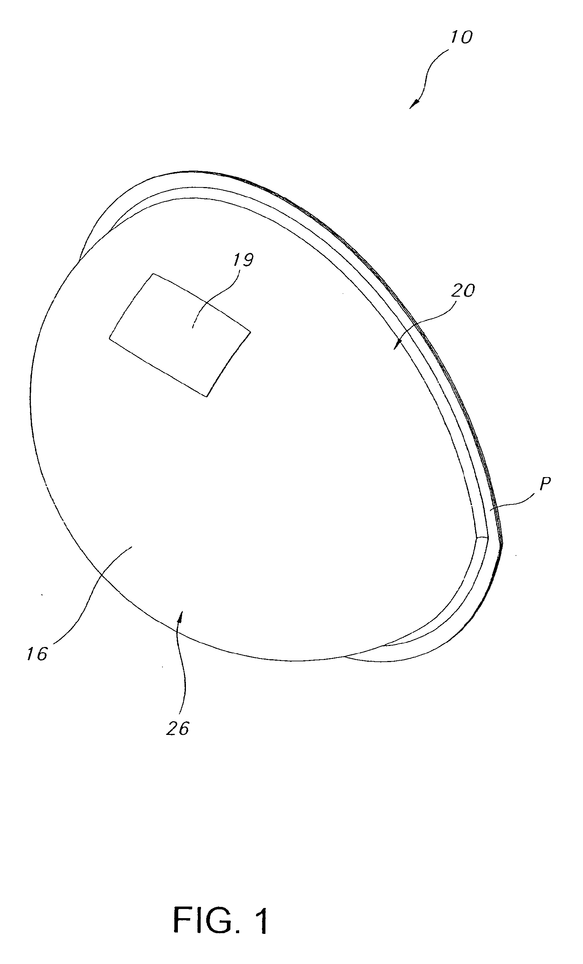

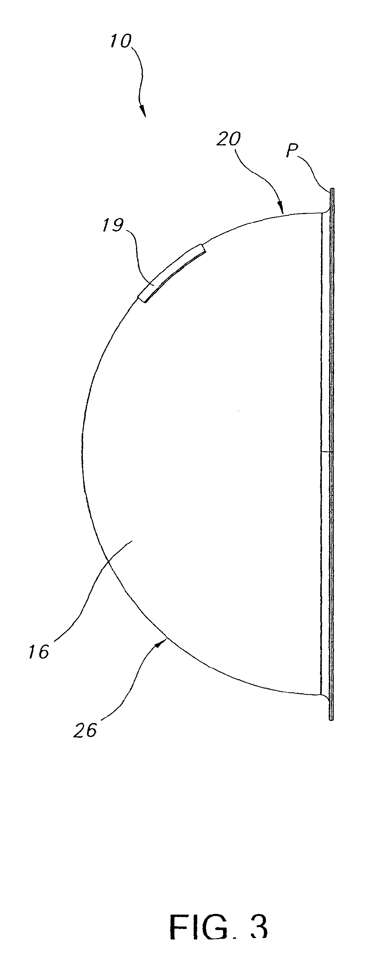

Referring to Figs. 1-5, a disposable absorbent article 10 in accordance with a first exemplary embodiment of the invention will be described. It should be pointed out that as used herein the term "disposable" means that article is designed to be used until soiled and then discarded, rather than being washed and used again.

In the exemplary embodiment of Figs. 1-5, the article 10 is in the form of a nursing pad. While the following description focuses on nursing pads, it should be clear that the subject invention can be used for any type of pad-like absorbent article to be worn by a person within an undergarment for trapping fluid.

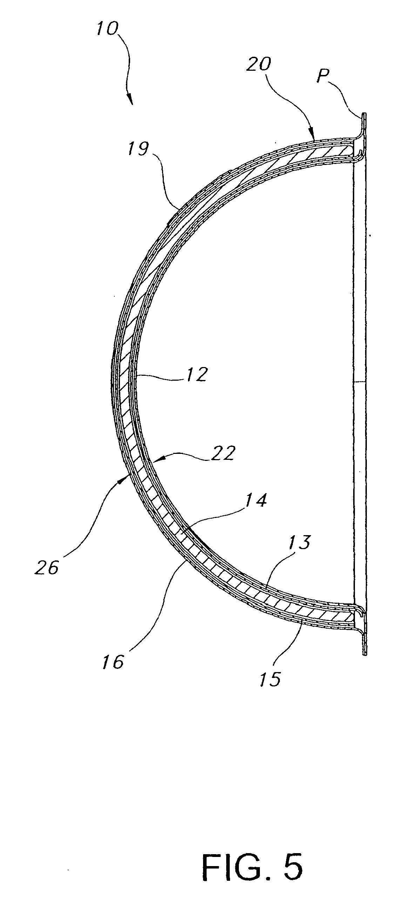

Referring to the figures, and most particularly Fig. 5, the absorbent article 10 is generally formed of a liquid pervious inner liner or layer 12, a liquid absorbent, e.g., air-laid composite, core 14, and an outer cover or moisture barrier 16. The inner layer 12 may be of any liquid pervious material and may be selected from a variety of textile-like films and fabrics. One particularly suitable material is a 20 gsm wettable nonwoven coverstock, made of spun bond polypropylene. In the present embodiment, a tissue layer 13, for example, a 20 gsm tissue paper, is provided between the inner layer 12 and the absorbent core 14. Alternatively, the inner layer 12 may be disposed directly on-top of the absorbent core 14. The inner layer 12 may be formed of other material fibers (e.g., polyethylene, bi-component, polyester, rayon, cotton, etc.), fiber combinations (e.g., spunbond, air laid, wet laid, carded, hydroentangled, etc.), and basis weights may be used as well. In fact, if desired, the inner layer 12 may be formed of a liquid impermeable material, e.g., three dimensional polymeric film, having plural apertures or pores extending therethrough so as to make the material liquid permeable.

The outer layer or moisture barrier 16 is disposed over the other side of the absorbent core 14, i.e., on the opposite side from the inner layer 12. In the present embodiment, a tissue layer 15, for example, a 20 gsm tissue paper, is provided between the outer layer 16 and the absorbent core 14. Alternatively, the outer layer 16 may be disposed directly on-top of the absorbent core 14. The outer layer 16 may be of any suitable generally liquid impervious material, for example, a nonwoven material with a polymer film applied thereto.

The core 14 can be made up of any suitable absorbent material, as well as combinations of different types of absorbent materials and may have any desired material weight and density. For example, in one preferred embodiment shown herein the absorbent core 14 is formed of an air-laid absorbent material, such as wood pulp, and a super absorbent polymer powder (SAP) and a binder. Examples of SAP include polyacrylamides, polyvinyl alcohol, polyacrylates, various grafted starches, and the like.

The inner layer 12 and the moisture barrier 16 are generally the same size and are disposed coincident with each other. The core 14 is of the same general shape as the inner layer 12 and the moisture barrier 16, but is slightly smaller in size, and is centered within the confines of the coincident peripheries of the inner layer 12 and moisture barrier 16. The marginal portions of the inner layer 12 and the moisture barrier 16 which extend beyond the periphery of the core 14 are secured together along their respective inner surfaces by any suitable means, e.g., thermal or ultrasonic bonding, or by an adhesive (not shown) such that the absorbent article 10 has an absorbent assembly 20 generally defined by the inner layer 12, the absorbent core 14 and the barrier 16.

Referring to Figs. 1, 3 and 5, the absorbent assembly 20 is formed with a cupped configuration such that the inner layer 12 defines a concave inner surface 22 and the moisture barrier 16 defines a convex outer surface 26. It is desirable that the absorbent assembly 20 is formed such that the cupped configuration is the absorbent article's 10 natural shape, i.e., the shape the article 10 assumes without any external force applied thereto. Such a natural cupped configuration may be obtained by forming the assembled absorbent assembly 20 to have a cupped configuration. Alternatively, the core 14 may be shaped to have a cupped configuration, and thereafter, assembled with the inner layer 12 and the moisture barrier 16 to form an absorbent assembly 20 with a cupped configuration. Various compression and/or molding techniques may be utilized to form the core 14 or the assembled absorbent assembly 20 with the cupped configuration. By forming the absorbent assembly 20 to define the cupped configuration, either directly or via forming of the core 14, the absorbent assembly 20 provides a substantially smooth radial surface. Such generally avoids the wrinkles, bunching and other imperfections which may occur and show through the wearer's clothing if the absorbent article 10 is shaped after manufacture.

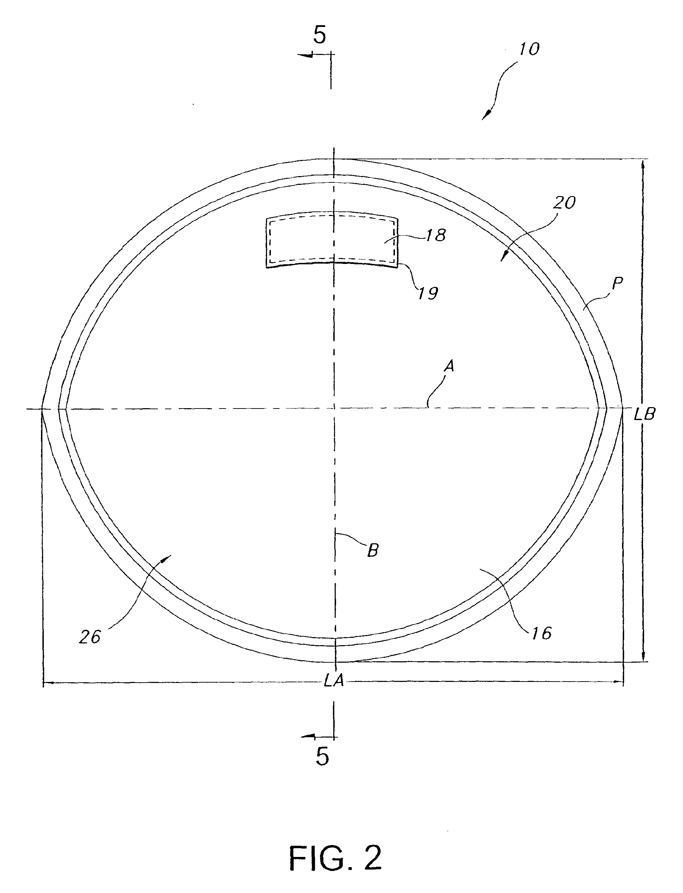

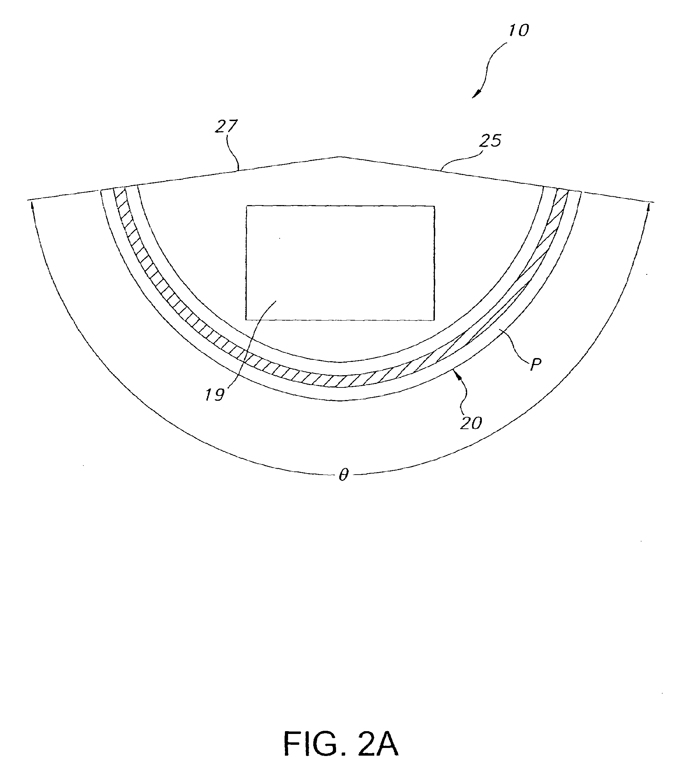

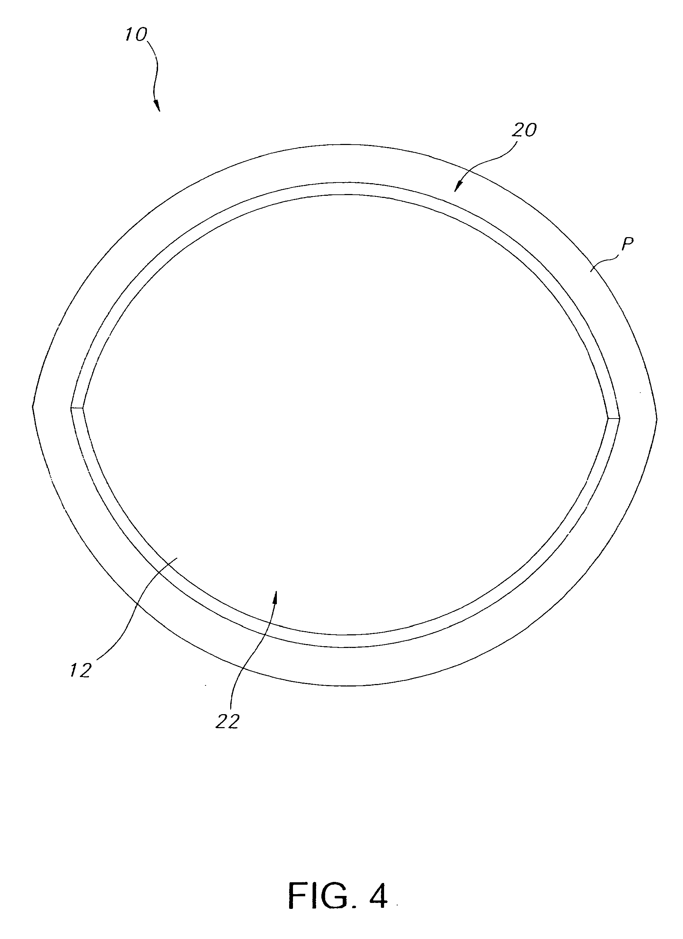

Referring to Figs. 2 and 4, the absorbent assembly 20 of the absorbent article 10 defines a perimeter P having a non-circular configuration. In the present embodiment, the perimeter P has an elliptical or "lemon shaped" configuration with a major axis A and a minor axis B. The ratio of the major axis length LA to the minor axis length LB is preferably between approximately 1.05:1 and 1.50:1, with a most preferred ratio of approximately 1.19:1. Other ratios outside of this range may also be utilized. An exemplary product having a major axis length LA of 125 mm and a minor axis length LB of 105 mm has been found to provide a desirable shape.

Referring to Fig. 2A, the absorbent article 10 is shown folded in half about the major axis A. As shown therein, the cupped configuration of the absorbent assembly 20 and the elliptical shape of the perimeter P define folded edges 25 and 27 which extend at an obtuse angle θ relative to one another. The angle θ is preferably in the range of approximately 150° and 175° and most preferably not more than approximately 175°, however, other angles are also possible.

The absorbent article 10 of the present embodiment provides a nursing pad which generally fits within a wearer's brassiere and provides a smooth and discrete outer surface. While these aspects are achieved by the cupped core and the given perimeter configuration, the invention is not limited to the specific configuration shown or the aesthetic appearance thereof.

In the present embodiment, a positioning adhesive 18 (shown in phantom in Fig. 2) is provided on the outer surface 26 of the moisture barrier 16 to help maintain the absorbent article in position relative to the wearer's garment, however, such is not required. The positioning adhesive 18 is provided within the upper hemisphere of the absorbent article 10, but may be otherwise positioned. Any suitable positioning adhesive can be used, such as a pressure sensitive hot melt adhesive. In order to protect the positioning adhesive 18 from degradation or being soiled by debris, a release strip 19 (e.g., a release paper) is releasably secured over the positioning adhesive 18. The release strip 19 can be formed of any suitable adhesive protective, yet easy to release, material.

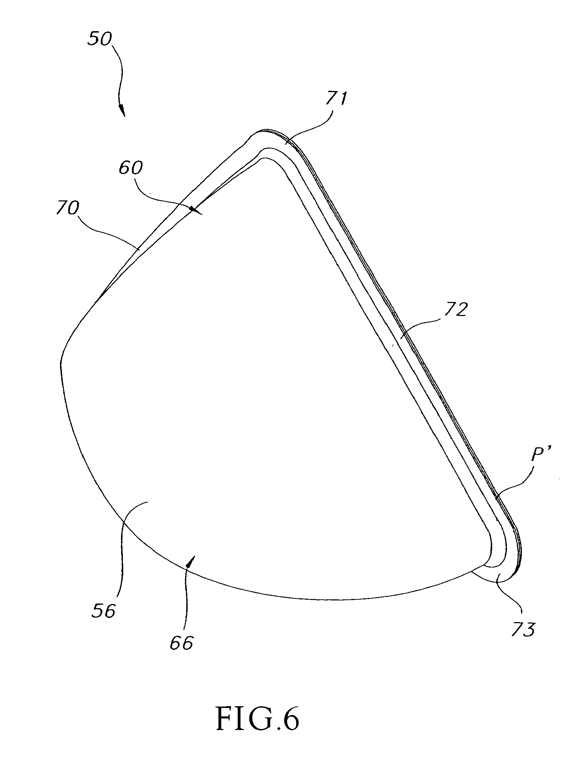

Referring to Figs. 6-9, a disposable absorbent article 50 in accordance with an alternative exemplary embodiment will be described. The article 50 is again shown and described in the form of a nursing pad although it can be used for any type of pad-like absorbent article to be worn by a person within an undergarment for trapping fluid.

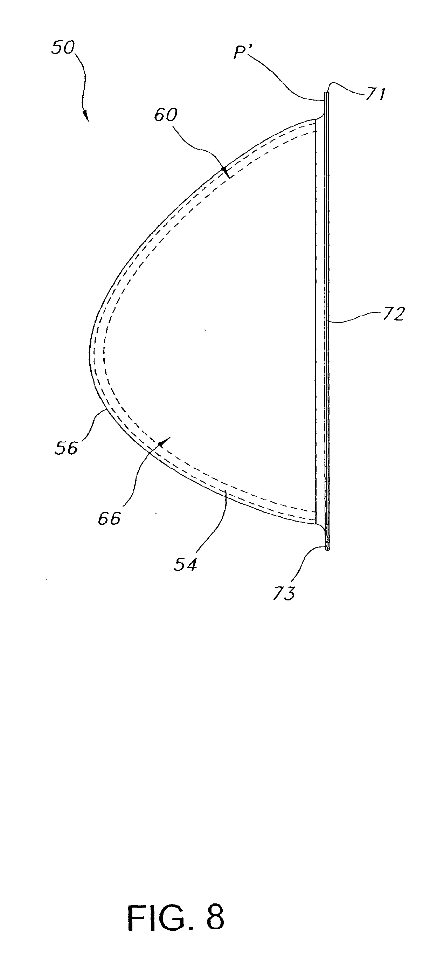

The absorbent article 50 is generally formed of a liquid pervious inner liner or layer 52, a liquid absorbent core 54 (shown in phantom in Fig. 8), and an outer cover or moisture barrier 56 similar to the previous embodiment. The materials and construction are generally the same as in the previous embodiment.

As in the previous embodiment, the inner layer 52 and the moisture barrier 56 are generally the same size and are disposed coincident with each other. The core 54 is of the same general shape as the inner layer 52 and the moisture barrier 56, but is slightly smaller in size, and is centered within the confines of the coincident peripheries of the inner layer 52 and moisture barrier 56. The marginal portions of the inner layer 52 and the moisture barrier 56 which extend beyond the periphery of the core 54 are secured together along their respective inner surfaces by any suitable means, e.g., thermal or ultrasonic bonding, or by an adhesive (not shown) such that the absorbent article 50 has an absorbent assembly 60 generally defined by the inner layer 52, the absorbent core 54 and the barrier 56.

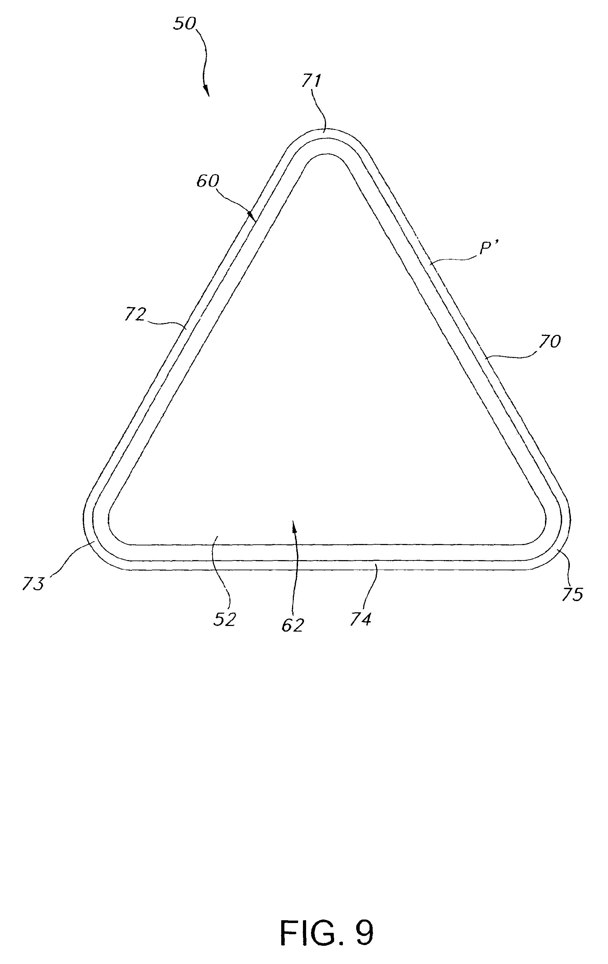

Referring to Figs. 6 and 8, the absorbent assembly 60 is again formed with a cupped configuration such that the inner layer 52 defines a concave inner surface 62 (see Fig. 9) and the moisture barrier 56 defines a convex outer surface 66. It is desirable that the absorbent assembly 60 is formed such that the cupped configuration is the absorbent article's 50 natural shape, i.e. the shape the article 50 assumes without any external force applied thereto. Such a natural cupped configuration may be obtained by forming the assembled absorbent assembly 60 to have a cupped configuration. Alternatively, the core 54 may be shaped to have a cupped configuration, and thereafter, assembled with the inner layer 52 and the moisture barrier 56 to form an absorbent assembly 60 with a cupped configuration. Again, various compression and/or molding techniques may be utilized to form the core 54 or the assembled absorbent assembly 60 with the cupped configuration. By forming the absorbent assembly 60 to define the cupped configuration, either directly or via forming of the core 14, the absorbent assembly 60 provides a substantially smooth radial surface.

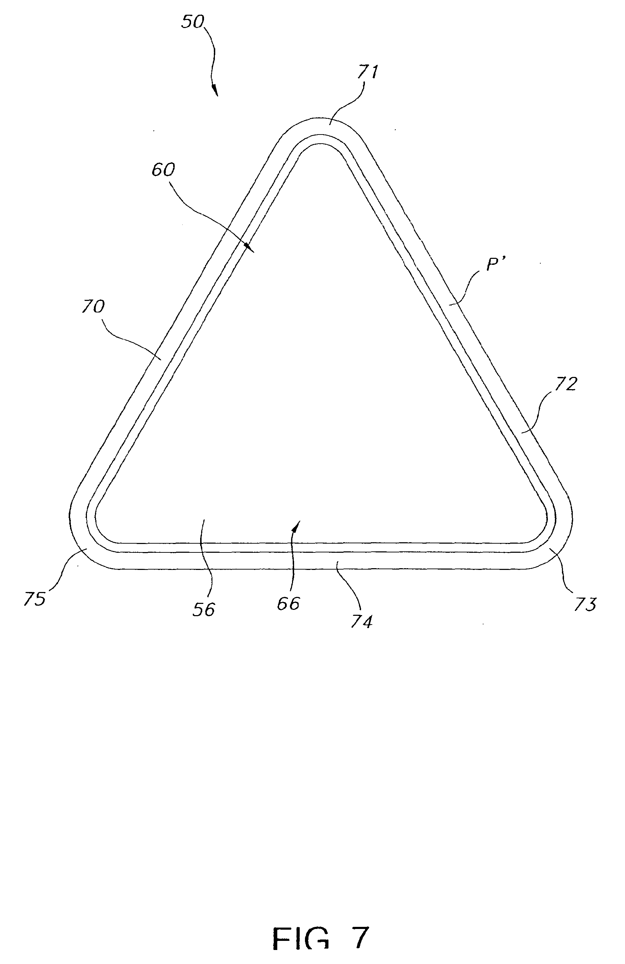

Referring to Figs. 7 and 9, the absorbent assembly 60 of the absorbent article 50 defines a perimeter P' having a non-circular configuration. In the present embodiment, the perimeter P' has a triangular configuration with three sides 70, 72, 74. In the illustrated embodiment, the three sides 70, 72 and 74 are equal in length such that the perimeter P' has an equilateral triangular configuration, however, such is not required. The sides 70, 72 and 74 intersect to define three vertices 71, 73 and 75. In the illustrated embodiment, each of the vertices 71, 73 and 75 has a rounded configuration, however, such is not required. One, two or none of the vertices 71, 73 and 75 may have such rounded configuration.

The present embodiment is not illustrated with a positioning adhesive, but may incorporate such.

The absorbent article 50 of the present embodiment provides a nursing pad which generally fits within a wearer's brassiere and provides a smooth and discrete outer surface. While these aspects are achieved by the cupped core and the given perimeter configuration, the invention is not limited to the specific configuration shown or the aesthetic appearance thereof.

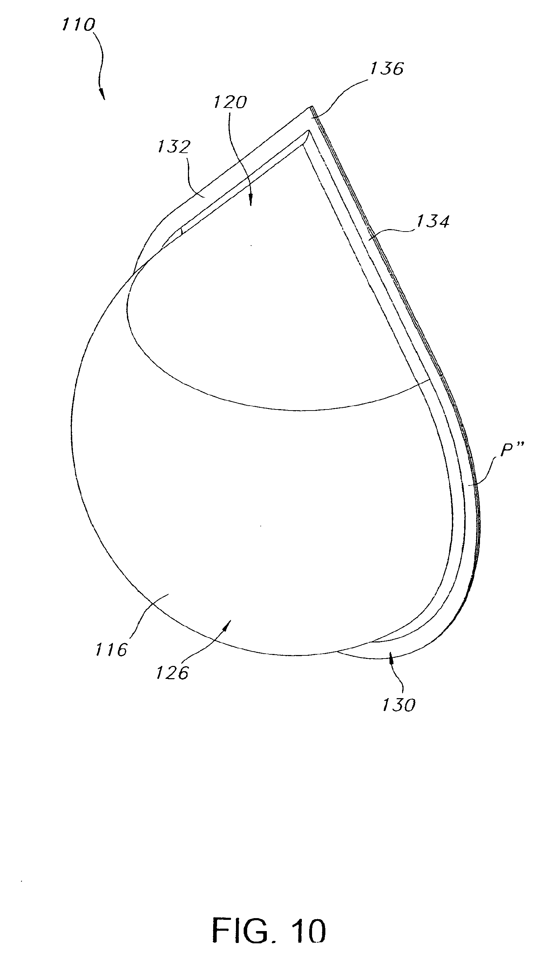

Referring to Figs. 10-13, a disposable absorbent article 110 in accordance with another alternative exemplary embodiment will be described. The article 110 is again shown and described in the form of a nursing pad although it can be used for any type of pad-like absorbent article to be worn by a person within an undergarment for trapping fluid.

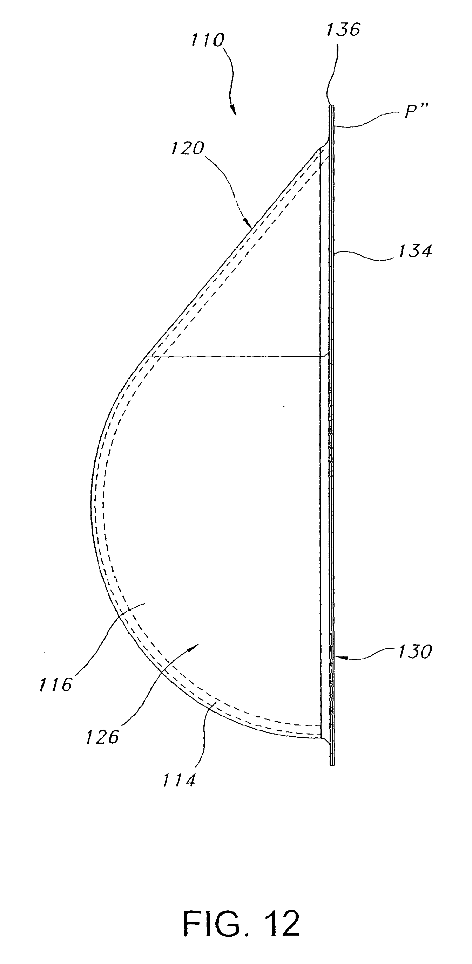

The absorbent article 110 is generally formed of a liquid pervious inner liner or layer 112, a liquid absorbent core 114 (shown in phantom in Fig. 12), and an outer cover or moisture barrier 116 similar to the previous embodiments. The materials and construction are generally the same as in the previous embodiments.

As in the previous embodiments, the inner layer 112 and the moisture barrier 116 are generally the same size and are disposed coincident with each other. The core 114 is of the same general shape as the inner layer 112 and the moisture barrier 116, but is slightly smaller in size, and is centered within the confines of the coincident peripheries of the inner layer 112 and moisture barrier 116. The marginal portions of the inner layer 112 and the moisture barrier 116 which extend beyond the periphery of the core 114 are secured together along their respective inner surfaces by any suitable means, e.g., thermal or ultrasonic bonding, or by an adhesive (not shown) such that the absorbent article 110 has an absorbent assembly 120 generally defined by the inner layer 112, the absorbent core 114 and the barrier 116.

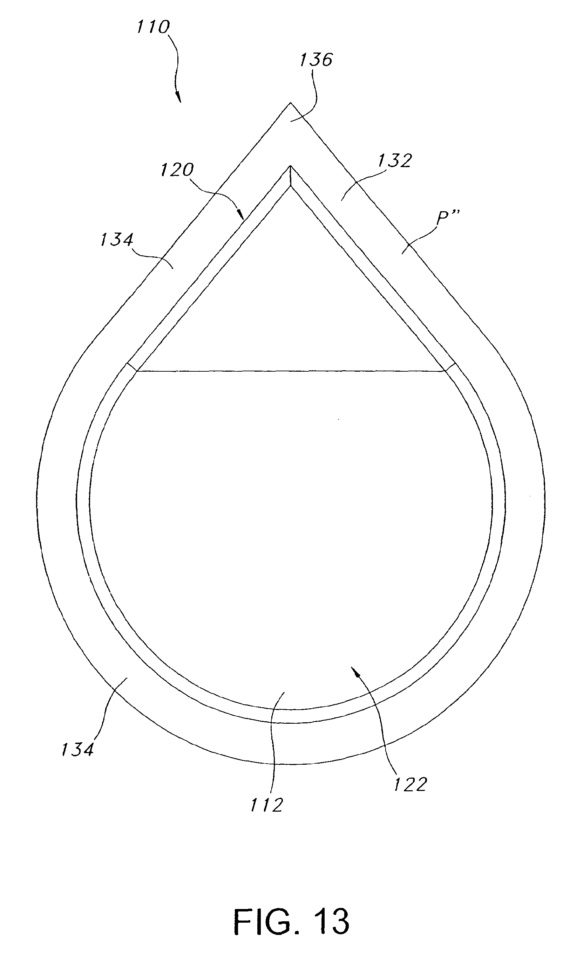

Referring to Figs. 10 and 12, the absorbent assembly 120 is again formed with a cupped configuration such that the inner layer 112 defines a concave inner surface 122 (see Fig. 13) and the moisture barrier 116 defines a convex outer surface 126. It is desirable that the absorbent assembly 120 is formed such that the cupped configuration is the absorbent article's 110 natural shape, i.e. the shape the article 110 assumes without any external force applied thereto. Such a natural cupped configuration may be obtained by forming the assembled absorbent assembly 120 to have a cupped configuration. Alternatively, the core 114 may be shaped to have a cupped configuration, and thereafter, assembled with the inner layer 112 and the moisture barrier 116 to form an absorbent assembly 120 with a cupped configuration. Again, various compression and/or molding techniques may be utilized to form the core 114 or the assembled absorbent assembly 120 with the cupped configuration. By forming the absorbent assembly 120 to define the cupped configuration, either directly or via forming of the core 14, the absorbent assembly 120 provides a substantially smooth radial surface.

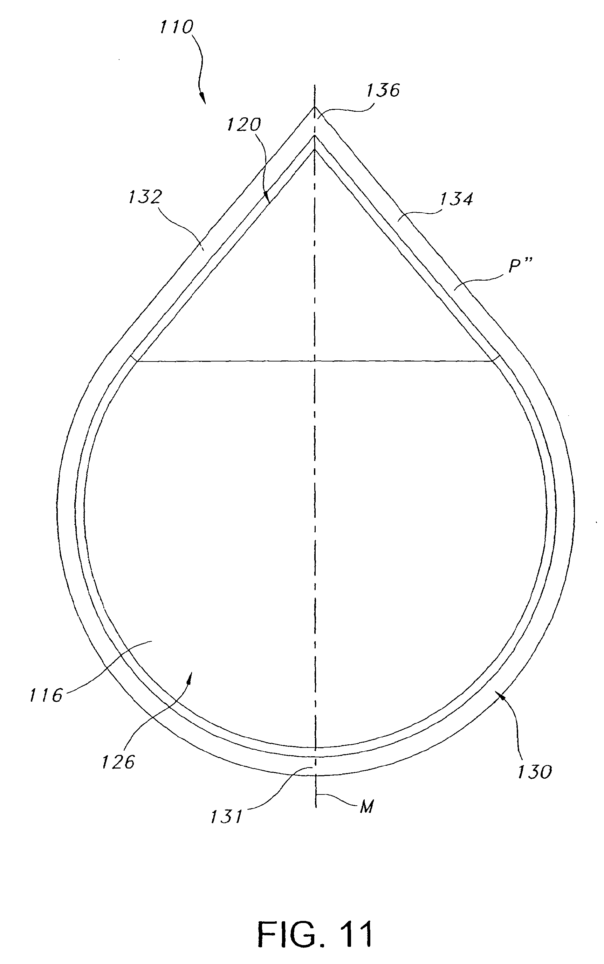

Referring to Figs. 11 and 13, the absorbent assembly 120 of the absorbent article 110 defines a perimeter P" having a non-circular configuration. In the present embodiment, the perimeter P" has a teardrop configuration with an arcuate portion 130 and opposed sides 132, 134 which taper from the arcuate portion to a point 136. In the illustrated embodiment, the absorbent assembly 120 is symmetrical about an axis M extending between the point 136 and a midpoint 131 along the arcuate portion 130, however, such is not required. The absorbent article 110 is generally not symmetrical about the axis perpendicular thereto, i.e. the side to side axis, but instead provides a larger concave portion in the lower hemisphere of the article 110.

The present embodiment is not illustrated with a positioning adhesive, but may incorporate such.

The absorbent article 110 of the present embodiment provides a nursing pad which generally fits within a wearer's brassiere and provides a smooth and discrete outer surface. While these aspects are achieved by the cupped core and the given perimeter configuration, the invention is not limited to the specific configuration shown or the aesthetic appearance thereof.

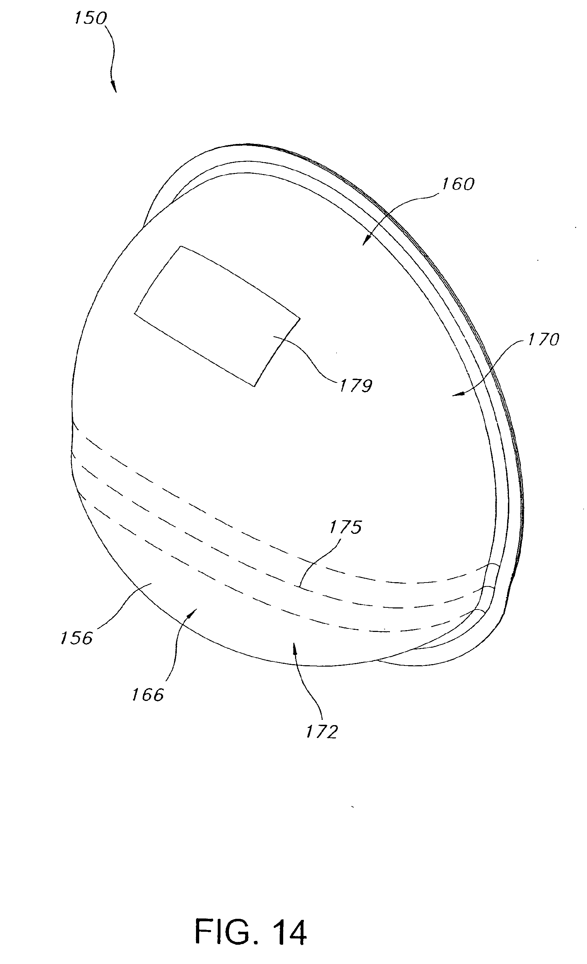

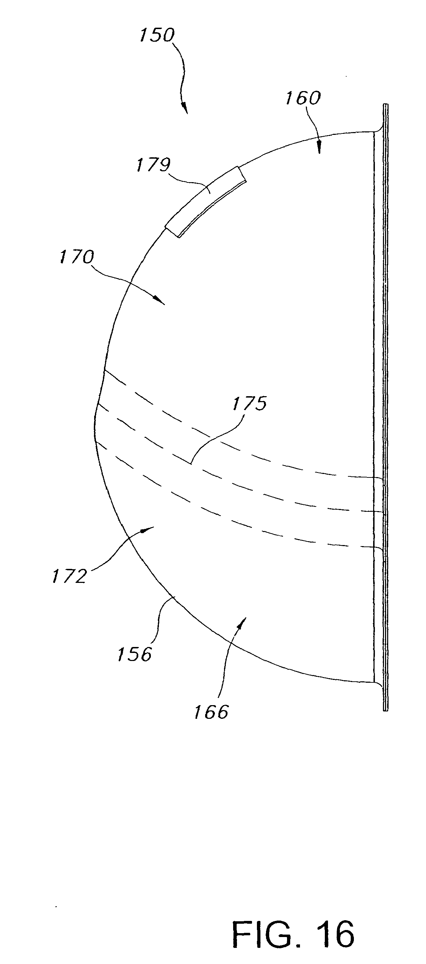

Referring to Figs. 14-18, a disposable absorbent article 150 in accordance with an alternative exemplary embodiment will be described. The article 150 is again shown and described in the form of a nursing pad although it can be used for any type of pad-like absorbent article to be worn by a person within an undergarment for trapping fluid.

The absorbent article 150 is generally formed of a liquid pervious inner liner or layer 152, a liquid absorbent core 154, and an outer cover or moisture barrier 156 similar to the previous embodiments. The materials and construction are generally the same as in the previous embodiments.

As in the previous embodiments, the inner layer 152 and the moisture barrier 156 are generally the same size and are disposed coincident with each other. The core 154 is of the same general shape as the inner layer 152 and the moisture barrier 156, but is slightly smaller in size, and is centered within the confines of the coincident peripheries of the inner layer 152 and moisture barrier 156. The marginal portions of the inner layer 152 and the moisture barrier 156 which extend beyond the periphery of the core 154 are secured together along their respective inner surfaces by any suitable means, e.g., thermal or ultrasonic bonding, or by an adhesive (not shown) such that the absorbent article 150 has an absorbent assembly 160 generally defined by the inner layer 152, the absorbent core 154 and the barrier 156.

Referring to Figs. 14, 16 and 18, the absorbent assembly 160 is again formed with a cupped configuration such that the inner layer 152 defines a concave inner surface 162 and the moisture barrier 156 defines a convex outer surface 166. It is desirable that the absorbent assembly 160 is formed such that the cupped configuration is the absorbent article's 150 natural shape, i.e. the shape the article 150 assumes without any external force applied thereto. Such a natural cupped configuration may be obtained by forming the assembled absorbent assembly 160 to have a cupped configuration. Alternatively, the core 154 may be shaped to have a cupped configuration, and thereafter, assembled with the inner layer 152 and the moisture barrier 156 to form an absorbent assembly 160 with a cupped configuration. Again, various compression and/or molding techniques may be utilized to form the core 154 or the assembled absorbent assembly 160 with the cupped configuration. By forming the absorbent assembly 160 to define the cupped configuration, either directly or via forming of the core 14, the absorbent assembly 160 provides a substantially smooth radial surface.

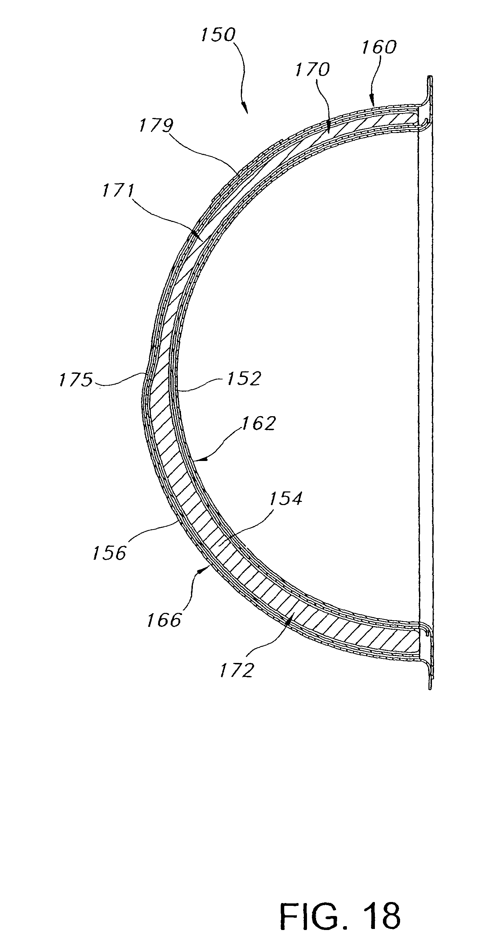

In the present embodiment, the compression and/or forming of the core 154 is controlled such that the core 154 defines areas having differing core thicknesses. Referring to Fig. 18, the upper hemisphere 170 of the core 154 is more compressed than the lower hemisphere 172. As a result, the upper hemisphere 170 defines a slimmer area 171 within the perimeter of the article 150 that has a core material thickness which is less than the core material thickness in the remainder of the core 154. For example, in the illustrated embodiment, the thickness of the core 154 in the lower hemisphere 172 is approximately twice the thickness of the core 154 in the upper hemisphere 170. This configuration provides greater absorbency in the lower hemisphere 172, where it is generally more needed, and provides a slimmer profile in the upper hemisphere 170. In the presently illustrated embodiment, the core thickness ratio is preferably not more than about 2:1, however, other ratios may also be utilized.

Furthermore, while the slimmer area 171 may be achieved by compressing the core material therein, the invention is not limited to such. For example, the slimmer area 171 may alternatively be achieved by providing less core material within the slimmer area 171. In the illustrated embodiment with a 2:1 core thickness ratio, for example, the slimmer area 171 may be provided with approximately one-half the amount of core material. Other methods of acheiving the different core thicknesses may also be utilized.

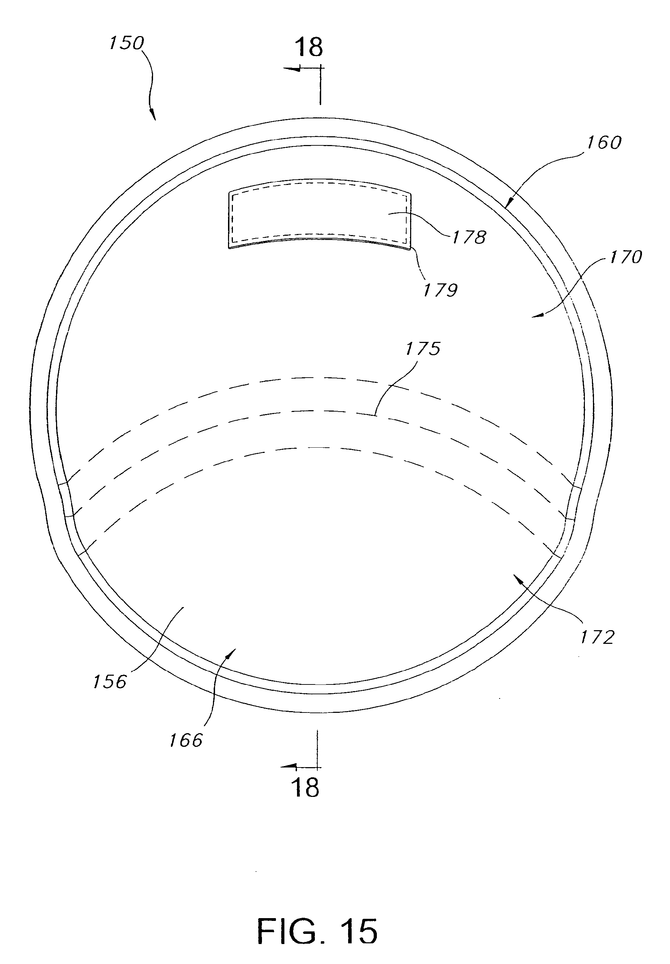

Referring to Fig. 15, the upper and lower hemispheres 170 and 172 are divided along one or more arcuate contour lines 175. Arcuate contour line 175 has a curvature approximately equal to the curvature of the absorbent assembly perimeter. The lower edge of the slimmer area 171 preferably follows the arcuate contour line 175 dividing the hemispheres 170 and 172. Other configurations may also be provided.

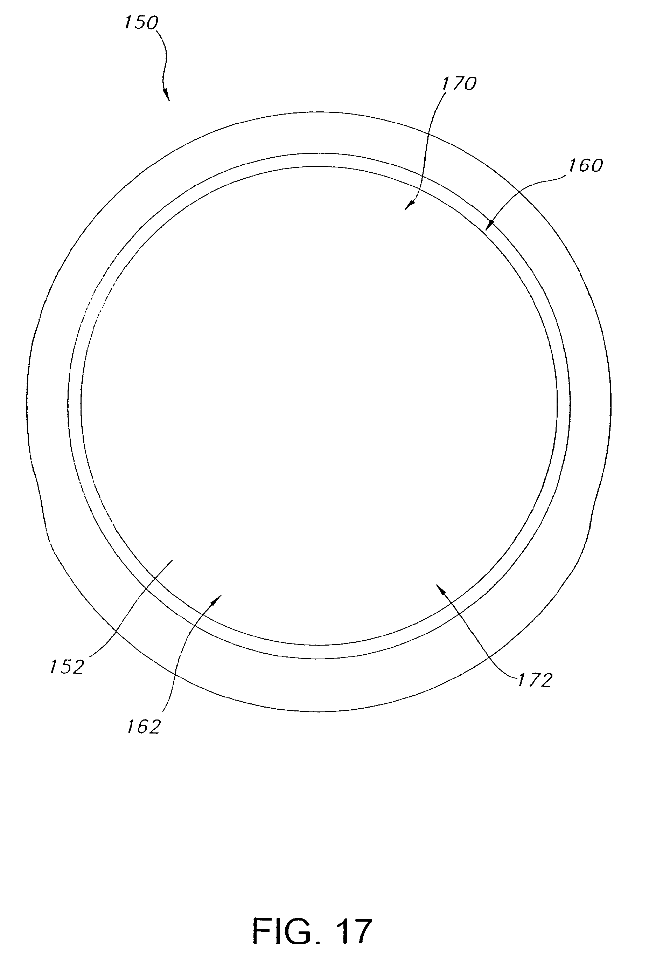

Referring to Figs. 15 and 17, it is noted that the absorbent assembly 160 of the absorbent article 150 has a generally circular perimeter. It is further noted that in the present embodiment, a positioning adhesive 178 (shown in phantom in Figure 15) and a release strip 179 are provided, but such are not required.

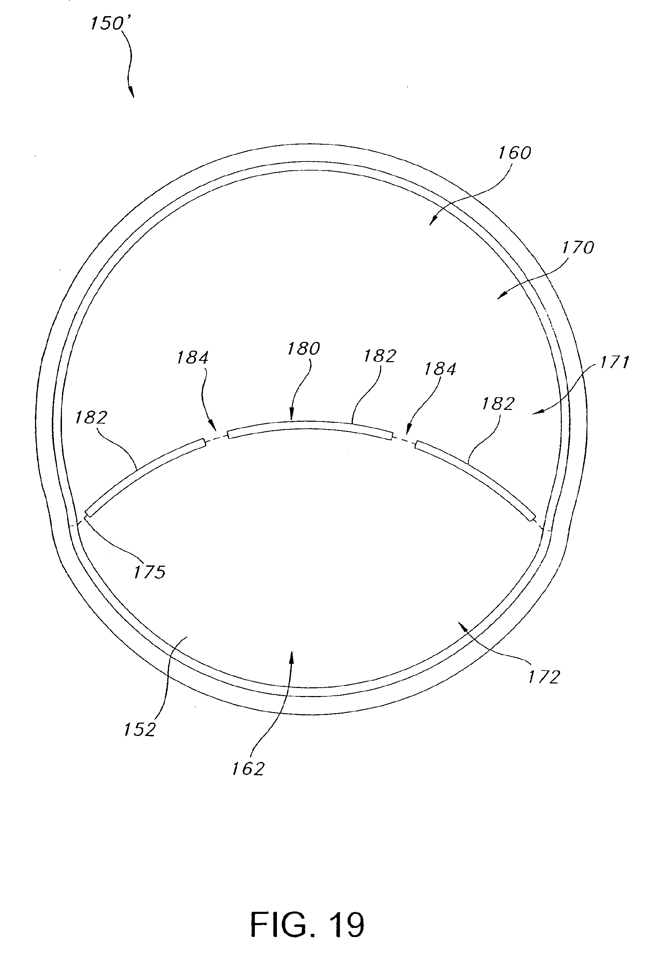

Referring to Fig. 19, a disposable absorbent article 150' in accordance with another alternative exemplary embodiment will be described. The article 150' is substantially the same as the absorbent article 150 illustrated in Figs. 14-18, but includes an embossing line 180 along the lower edge of the slimmer area 171.

Each section 182 of each embossing line 180 is produced by applying pressure and/or heat to the portions of the article 150' along the line to compress and increase the density of the materials along those lines. The compression and/or heat applied to the materials making up the core 154 causes the interstitial space between the individual fibers making up the core to compress or become densified. Such densified areas may inhibit liquid to flow therethrough, and thereby assist in controlling fluid flow within the absorbent article 150'. The uncompressed sections 184 between the embossing line portions 182 may also be provided to further control fluid flow in a desired fashion. The application of pressure and heat can be accomplished using conventional thermal or ultrasonic bonding techniques or by pattern embossing. In some applications the use of pressure alone may be sufficient to produce a dense embossing line which remains after the pressure is removed. Moreover, an adhesive may be used when pressure is applied to create the dense embossing line 180. Since the embossing line 180 remains, the embossing line 180 helps to further define the shape of the assembled absorbent article 150'. While only one embossing line 180 is shown, multiple embossing lines 180 may be provided, for example, along the various contour lines 175.

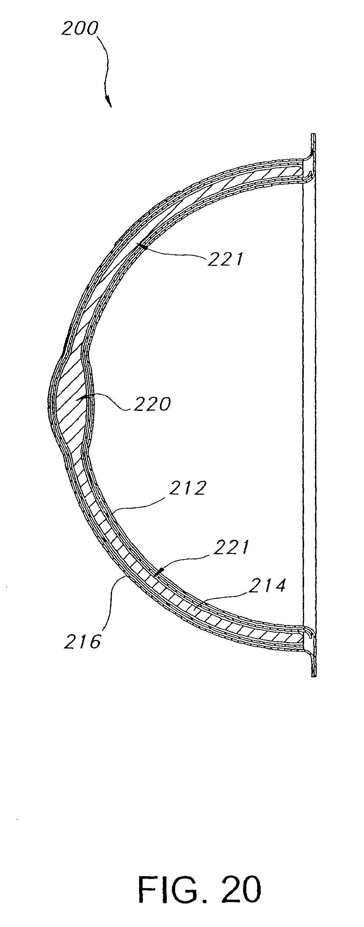

Referring to Fig. 20, a disposable absorbent article 200 in accordance with an alternative exemplary embodiment will be described. The article 200 is substantially similar to the article 150 illustrated in Figs. 14-18 and generally includes a liquid pervious inner liner or layer 212, a liquid absorbent core 214, and an outer cover or moisture barrier 216 similar to the previous embodiments.

As in the embodiment illustrated in Figs. 14-18, the compression and/or forming of the core 214 is controlled such that the core 214 defines areas having differing core thicknesses. Referring to Fig. 20, the center area 220 of the core 214 is less compressed than the slimmer area 221 extending circumferentially about the center area 220. As a result, the slimmer area 221 has a core material thickness which is less than the core material thickness in the center area 220 of the core 214. For example, in the illustrated embodiment, the thickness of the center area 220 of the core 214 is approximately twice the thickness of the remainder of the core 214 in the slimmer area 221. This configuration provides greater absorbency in the center area 220, where it is generally more needed, and provides a slimmer profile in the slimmer area 221. Again, while an approximately 2:1 thickness ratio is illustrated, other ratios may be utilized.

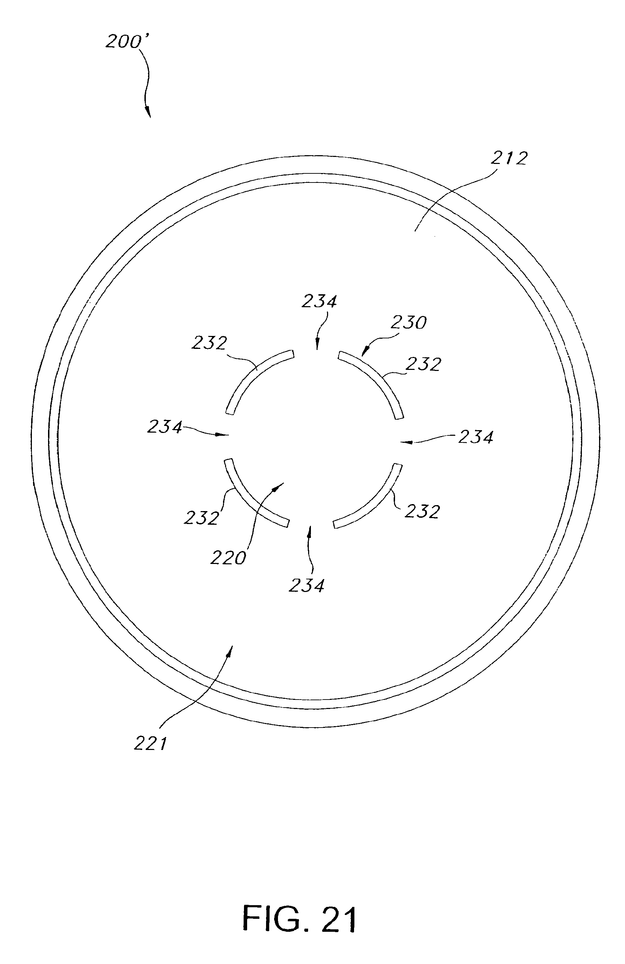

Referring to Fig. 21, a disposable absorbent article 200' in accordance with another alternative exemplary embodiment will be described. The article 200' is substantially the same as the absorbent article 200 illustrated in Fig. 20, but includes a circumferential embossing line 230 about the center area 220.

As explained above, each section 232 of embossing line 230 is produced by applying pressure and/or heat to the portions of the article 200' along the line to compress and increase the density of the materials along those lines. The uncompressed sections 234 between the embossing line portions 232 may be provided to allow fluid to flow from the center area 220 to the slimmer area 221 in a controlled manner if desired. Since the embossing line 230 remains, the embossing line 230 helps to further define the shape of the assembled absorbent article 200'.

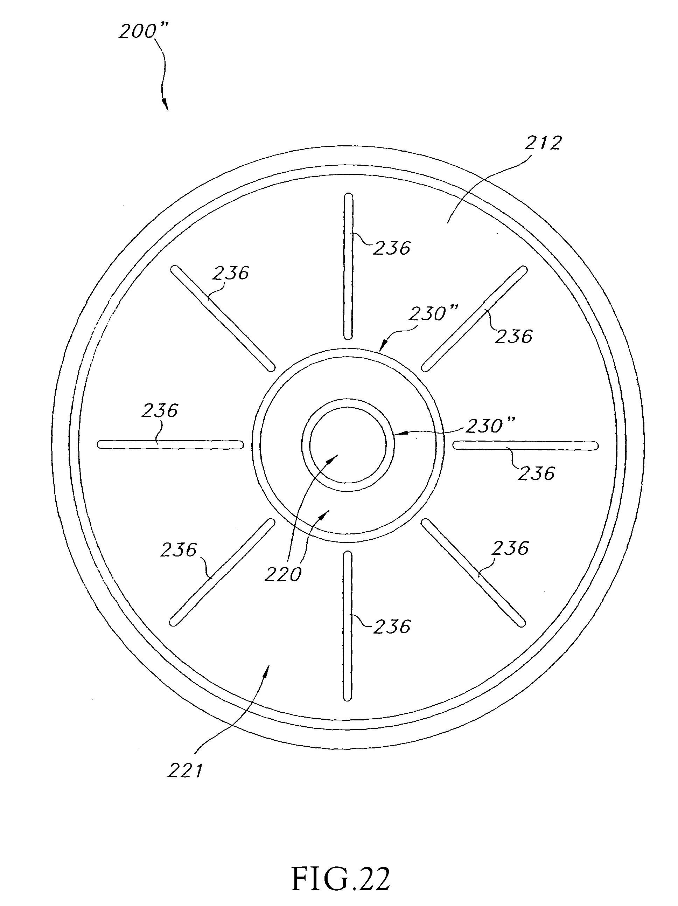

Referring to Fig. 22, a disposable absorbent article 200" in accordance with another alternative exemplary embodiment will be described. The article 200" is substantially the same as the absorbent article 200' illustrated in Fig. 21, but includes concentric continuous embossing lines 230" about the center area 220. Additionally, the absorbent article 200" includes one or more radial embossing lines 236 which extend radially from the center area 220 toward the perimeter. While the radial embossing lines 236 are illustrated in conjunction with the circumferential embossing lines 230", they may also be provided alone, without circumferential embossing lines. The embossing lines 230" and 236 again help to define the shape of the assembled absorbent article 200".

While preferred embodiments of the invention have been shown and described herein, it will be understood that such embodiments are provided by way of example only. Numerous variations, changes and substitutions will occur to those skilled in the art without departing from the spirit of the invention. Accordingly, it is intended that the appended claims cover all such variations as fall within the spirit and scope of the invention.

## Claims

1. A nursing pad comprising:
an absorbent assembly having an inner surface and an outer surface with an absorbent material therebetween, the inner and outer surfaces connected about a perimeter of the absorbent assembly, wherein the absorbent assembly perimeter has a non-circular shape and the absorbent assembly has a cupped configuration such that the inner surface is concave and the outer surface is convex.

2. The nursing pad according to claim 1 wherein the absorbent assembly perimeter has an elliptical shape with a major and minor axis.

3. The nursing pad according to claim 2 wherein a ratio of the major axis to the minor axis is between approximately 1.05:1 and 1.50:1.

4. The nursing pad according to claim 2 wherein a ratio of the major axis to the minor axis is approximately 1.19:1.

5. The nursing pad according to claim 1 wherein the absorbent assembly perimeter has a shape with an arcuate portion and opposed side portions which taper from the arcuate portion to a point.

6. The nursing pad according to claim 5 wherein the absorbent assembly perimeter is substantially symmetrical about a plane extending between the point and a midpoint of the arcuate portion.

7. The nursing pad according to claim 1 wherein the absorbent assembly perimeter has a generally triangular shape.

8. The nursing pad according to claim 7 wherein the absorbent assembly perimeter includes three equilateral sides.

9. The nursing pad according to claim 7 wherein the absorbent assembly perimeter defines three vertices, each of the vertices having a rounded configuration.

10. The nursing pad according to claim 1 wherein the absorbent assembly is formed to have a cupped configuration with a substantially smooth radial surface after assembly thereof.

11. The nursing pad according to claim 1 wherein the absorbent material is formed to have a cupped configuration with a substantially smooth radial surface prior to assembly of the absorbent assembly.

12. A nursing pad comprising:
an absorbent assembly having an inner surface and an outer surface which are connected about a perimeter of the absorbent assembly; and
an absorbent pulp material provided between the inner and outer surfaces within the absorbent assembly perimeter, the absorbent pulp material having a reduced material thickness in at least one slimmer area within the absorbent assembly perimeter that is less than a total area within the absorbent assembly perimeter such that at least two areas having pulp material of differing thickness are defined.

13. The nursing pad according to claim 12 wherein the absorbent assembly has a center area within the absorbent assembly perimeter and the at least one slimmer area extends circumferentially about the center area such that the center area has a pulp thickness which is greater than the pulp thickness of the remainder of the area within the absorbent assembly perimeter.

14. The nursing pad according to claim 13 wherein at least one embossing line extends circumferentially about the center area between the center area and the slimmer area.

15. The nursing pad according to claim 13 wherein at least two concentric embossing lines extend circumferentially about the center area.

16. The nursing pad according to claim 13 wherein at least one embossing line extends radially from the center area toward the absorbent assembly perimeter.

17. The nursing pad according to claim 12 wherein the absorbent assembly has first and second substantial hemispheres and wherein the at least one slimmer area is in the first substantial hemisphere such that the first substantial hemisphere has a pulp thickness which is less than the pulp thickness in the second substantial hemisphere.

18. The nursing pad according to claim 17 wherein the first and second substantial hemispheres are divided by an arcuate contour line.

19. The nursing pad according to claim 18 wherein the first substantial hemisphere has an arcuate outer perimeter which has a radius approximately equal to a radius of the arcuate contour line.

20. The nursing pad according to claim 17 wherein at least one embossing line extends along the arcuate contour line between the first and second hemispheres.

21. The nursing pad according to claim 12 wherein the slimmer area and the remainder of the absorbent pulp material have a thickness ratio of approximately 1:2.

22. A nursing pad comprising:
an absorbent assembly having an inner surface and an outer surface with an absorbent material therebetween, the inner and outer surfaces connected about a perimeter of the absorbent assembly, wherein, in an unfolded condition, the absorbent assembly has a cupped configuration such that the inner surface is concave and the outer surface is convex, and, in a folded condition, the absorbent assembly defines opposed folded sides extending between the perimeter, the opposed folded sides extending at an angle relative to one another in the range of approximately 150° to 175°.

23. A nursing pad comprising:
an absorbent assembly having an inner surface and an outer surface with an absorbent material therebetween, the inner and outer surfaces connected about a perimeter of the absorbent assembly, wherein the absorbent assembly perimeter has an elliptical shape with a major and minor axis.

24. The nursing pad according to claim 23 wherein a ratio of the major axis to the minor axis is between approximately 1.05:1 and 1.50:1.

25. The nursing pad according to claim 23 wherein a ratio of the major axis to the minor axis is approximately 1.19:1.
